# EUROPÄISCHE PATENTANMELDUNG

(11) **EP 3 225 998 A1**
(43) Veröffentlichungstag der Anmeldung: **04.10.2017**
(21) Anmeldenummer: 16163255.9
(22) Anmeldetag: 31.03.2016
(51) Int. Cl.: G01N 33/86

(54) **VERFAHREN ZUR BESTIMMUNG VON FIBRINOGEN**

(71) Anmelder: Siemens Healthcare Diagnostics Products GmbH, 35041 Marburg (DE)
(72) Erfinder: Greis, Dirk, 35099 Burgwald (DE); Zander, Norbert, 35039 Marburg (DE)

(57) **Zusammenfassung**

Die vorliegende Erfindung liegt auf dem Blutgerinnungsdiagnostik und betrifft ein kinetisches Verfahren zur Bestimmung der Fibrinogenkonzentration in einer humanen Plasmaprobe gemäß der Clauss-Methode.

## Beschreibung

Die vorliegende Erfindung liegt auf dem Gebiet der Blutgerinnungsdiagnostik und betrifft ein Verfahren zur Bestimmung der Fibrinogenkonzentration in einer humanen Plasmaprobe gemäß der Clauss-Methode.

Fibrinogen ist die wasserlösliche Vorstufe von Fibrin, welches die Matrix für den Wundverschluss bildet. Die Gerinnungsprotease Thrombin (Faktor IIa) spaltet Fibrinogen und aktiviert auf diese Weise die Fibrinbildung, also die Gerinnselbildung. Erniedrigte Fibrinogenspiegel gehen mit einer Blutungsneigung einher. Akut erhöhte Fibrinogenspiegel findet man häufig bei Entzündungen, postoperativ und in anderen Situationen. Langfristig erhöhte Fibrinogenspiegel gelten als Risikoindikator für thrombotische Erkrankungen.

Im Stand der Technik sind eine Reihe verschiedener Methoden zur Bestimmung der Fibrinogenkonzentration bekannt.

Ein häufig angewandtes Verfahren zur Bestimmung der Fibrinogenkonzentration ist die sogenannte Clauss-Methode (Clauss, A., Gerinnungsphysiologische Schnellmethode zur Bestimmung des Fibrinogens. 1957, Acta haemat. 17: 237-246). Der Test ist eine Variation der Thrombinzeit, bei der eine Plasmaprobe mit Thrombin vermischt wird und die Gerinnungszeit bestimmt wird. Bei der Clauss-Methode wird im Reaktionsansatz eine verhältnismäßig geringe Fibrinogenkonzentration mit einer verhältnismäßig hohen, standardisierten Thrombinkonzentration kombiniert, wodurch die Fibrinbildungsgeschwindigkeit praktisch ausschließlich mit der Fibrinogenkonzentration korreliert. Die verhältnismäßig geringe Fibrinogenkonzentration im Reaktionsansatz wird üblicherweise durch die Verwendung von vorverdünnten Plasmaproben hergestellt.

Im Reaktionsansatz wird dann die Fibrinbildung, also die Gerinnselbildung photometrisch bestimmt. Durch die Fibrinbildung trübt sich der Reaktionsansatz zunehmend, so dass durch eine Absorptionsmessung die Fibrinbildung quantitativ gemessen werden kann.

Üblicherweise wird dann die Gerinnungszeit der Probe bestimmt. Die Gerinnungszeit der Probe verhält sich proportional zur Fibrinogenmenge. Die Gerinnungszeit einer Probe ist die Zeit vom Zeitpunkt der Zugabe von Thrombin zur Probe bis zum Zeitpunkt der Messung einer fassbaren Fibrinbildung, also Trübung des Reaktionsansatzes. Dabei kann die "fassbare Fibrinbildung" als ein test- und gerätespezifischer Schwellenwert definiert sein, der -wenn er überschritten wird- die Gerinnungszeit angibt. Alternativ kann die "fassbare Fibrinbildung" ausgehend von der Signaldifferenz vor dem Start und nach Abschluss der Gerinnungsreaktion als ein test- und gerätespezifischer prozentualer Signaldifferenzwert definiert sein, der -wenn er erreicht wurde- die Gerinnungszeit angibt.

Problematisch ist, dass durch die relativ hohe Verdünnung der Plasmaproben im Clauss-Test nur relativ niedrige Signalstärken erzielt werden, die durch den in optischen Systemen niedrigen Signal-Rausch-Abstand und bereits durch geringfügige Störungen, wie beispielsweise Gasbläschen im Reaktionsansatz, die durch die Verwendung gekühlter Reagenzien entstehen können, zu unpräzisen Messergebnissen führen können.

Um diese Probleme zu vermeiden, werden im Stand der Technik ungekühlte Reagenzien verwendet, und/oder es wird Kaolin als signalverstärkendes Zusatzreagenz zugegeben.

Die Verwendung ungekühlter Reagenzien hat den Nachteil, dass die Reagenzien eine verminderte Stabilität aufweisen und möglichst schnell aufgebraucht werden sollten. Außerdem sind in modernen Analysegeräten häufig ausschließlich gekühlte Aufbewahrungspositionen für Reagenzbehälter vorgesehen, so dass ohne Weiteres gar kein ungekühltes Reagenz vorgesehen werden kann. Die Verwendung signalverstärkender Zusatzreagenzien ist ebenfalls unerwünscht, einerseits aus wirtschaftlichen Gründen und andererseits, weil sich durch den zusätzlich erforderlichen Pipettierschritt die Abarbeitung des Tests verlängert. Darüber hinaus kann sich das bildende Gerinnsel besonders bei niedrigen Plasmaspiegeln von Fibrinogen zusammenballen und als "Fibrin-Kaolin-Blase" im Reaktionsansatz herumschwimmen.

Ferner ist es problematisch, dass bei der Bestimmung der Gerinnungszeit einer Probe idealerweise mit der Messung unmittelbar nach der Zugabe des Thrombins zur Probe begonnen werden sollte. In der Praxis vergeht jedoch bei automatisierter Durchführung auf Analysengeräten eine kurze Zeitspanne (mehrere Sekunden) zwischen der Zugabe des Thrombinreagenzes in das Reaktionsgefäß und dem Absetzen des Reaktionsgefäßes in die Messposition. Bei sehr hohen Plasmaspiegeln von Fibrinogen kann es aber während dieser kurzen Zeit bereits zu einem Start der Reaktion und damit zu einem Signalanstieg kommen. In diesem Fall ist eine klassische Bestimmung der Gerinnungszeit schwierig, da für die "fassbare Fibrinbildung", sowohl über einen test- und gerätespezifischen Schwellenwert oder alternativ über die prozentuale Signaldifferenz bezogen auf Start und Abschluss der Gerinnungsreaktion, der Startwert bekannt sein muss.

Die der Erfindung zugrunde liegenden Aufgabe besteht also darin, die Clauss-Methode zur Bestimmung der Fibrinogenkonzentration in einer humanen Plasmaprobe so zu modifizieren, dass eine automatische Abarbeitung mit hoher Präzision durchführbar ist und dass auf die Verwendung ungekühlter Reagenzien und den Einsatz signalverstärkender Zusatzreagenzien, wie beispielsweise Kaolin, verzichtet werden kann.

Die Aufgabe wird dadurch gelöst, dass nicht mehr die bisher gebräuchliche Gerinnungszeit der Probe bestimmt wird, sondern dass die Reaktionskinetik der Fibrinbildung gemessen wird und die Reaktionsgeschwindigkeit zur Fibrinogenkonzentrationsbestimmung verwendet wird.

Gegenstand der vorliegenden Anmeldung ist also ein Verfahren zur Bestimmung der Fibrinogenkonzentration in einer humanen Plasmaprobe gemäß der Clauss-Methode, das Verfahren umfassend die Schritte:
- Bereitstellen eines Reaktionsansatzes enthaltend die Plasmaprobe und Thrombin,
- Messen der Extinktionswerte des Reaktionssatzes über die Zeit,
- Ermitteln der maximalen Extinktionsänderung und
- Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.

Das Mischungsverhältnis von Plasmaprobe und Thrombineinheiten zur Bereitstellung eines Reaktionsansatzes gemäß der Clauss-Methode, d.h. die Kombination einer verhältnismäßig geringen Fibrinogenkonzentration (aus der Probe) mit einer verhältnismäßig hohen Thrombinkonzentration, so dass die Fibrinbildungsgeschwindigkeit praktisch ausschließlich mit der Fibrinogenkonzentration korreliert, ist dem Fachmann hinreichend bekannt (siehe auch Thomas, L., Labor und Diagnose, 7. Auflage, TH-Books Verlagsgesellschaft mbH, Frankfurt/Main, 2008, Kapitel 16.15.2).

Vorzugsweise enthält der Reaktionsansatz dazu etwa 0,03 bis 0,4 mg/mL Fibrinogen und etwa 25 bis 40 IU/mL Thrombin. Diese Konzentrationen können beispielsweise dadurch erhalten werden, dass eine ca. 1:10 Verdünnung einer zu untersuchenden Plasmaprobe in einem Puffer und ein gepuffertes Thrombinreagenz mit einer Konzentration von ca. 100 IU/mL Thrombin in einem Verhältnis von 2+1 gemischt werden. Damit können Fibrinogenkonzentrationen von etwa 0,5 g/L bis 6,0 g/L bestimmt werden. Durch Änderung der Vorverdünnung der Probe kann der gesamte physiologisch vorkommende Bereich von Fibrinogen im menschlichen Plasma gemessen werden.

Das Messen der Extinktionswerte des Reaktionssatzes über die Zeit kann photometrisch, d.h. durch die Messung der Lichtabschwächung eines durch den Reaktionsansatz gesendeten Lichtstrahls, oder nephelometrisch, d.h. durch die Messung von Streulichtanteilen eines durch den Reaktionsansatz gesendeten Lichtstrahls, erfolgen. Idealerweise wird mit der Messung unmittelbar nach der Zugabe des Thrombins zur Probe begonnen, und die Messung der Extinktionswerte wird kontinuierlich bis zum Abschluss der Fibrinbildung durchgeführt. In der Praxis vergeht jedoch bei automatisierter Durchführung auf Analysengeräten eine kurze Zeitspanne (mehrere Sekunden) zwischen der Zugabe des Thrombinreagenzes in das Reaktionsgefäß mit anschließendem Mischen und dem Absetzen des Reaktionsgefäßes in die Messposition.

Das Ermitteln der maximalen Extinktionsänderung kann die folgenden Schritte umfassen:
- Erstellen einer Reaktionskurve aus den gemessenen Extinktionswerten über die Zeit, und
- Durchführen eines Regressionsverfahrens.

Das Regressionsverfahren ist eine kinetische Auswertemethode, die dem Fachmann bekannt ist und bei der in Abhängigkeit von einer in einem ersten Auswertungsschritt grob ermittelten Steigung der Reaktionskurve in einem zweiten Auswertungsschritt ein individuelles und optimiertes Regressionsintervall für die Berechnung der maximalen Reaktionsgeschwindigkeit, also der maximalen Extinktionsänderung, bestimmt wird.

Dazu wird zunächst innerhalb eines test- und gerätespezifischen definierten Auswertebereichs beginnend ab dem ersten Messwert (der so nahe wie möglich am Zeitpunkt der Thrombinzugabe liegt) über ein test- und gerätespezifisches Regressionsintervall die Steigung (= Reaktionsgeschwindigkeit bzw. Extinktionsänderung) per Regression nach der Methode der kleinsten Quadrate berechnet. Diese Berechnung wird mehrmals wiederholt, indem der zweite, dritte, vierte usw. Messwert als Startpunkt des Regressionsintervalls verwendet wird.

Die so ermittelte maximale Steigung (Grobsteigung) dient zur Berechnung des endgültigen Regressionsintervalls über eine Potenzfunktion mit empirisch, vorab festgelegten Parametern Faktor und Exponent. Über einen negativen Exponenten wird die gewünschte Wirkung erzielt, dass mit wachsender Steigung das Regressionsintervall kürzer wird. Grundsätzlich kann die Berechnung des endgültigen Regressionsintervalls jedoch auch über eine andere Gleichung erfolgen oder einer in der Software hinterlegten Tabelle entnommen werden.

Mit dem berechneten, endgültigen Regressionsintervall wird die Ermittlung der endgültigen maximalen Steigung wie bereits oben beschrieben durchgeführt, wiederum beginnend ab dem ersten Messwert per Regression nach der Methode der kleinsten Quadrate. Diese Berechnung wird mehrmals wiederholt, indem der zweite, dritte, vierte usw. Messwert als Startpunkt des Regressionsintervalls verwendet wird.

Die Bestimmung der Fibrinogenkonzentration der Probe erfolgt nun mit Hilfe einer Kalibrationskurve, die aus einer Zuordnung von bekannten Fibrinogenkonzentrationen und maximaler Steigung (= maximale Extinktionsänderung) erstellt wurde.

Ein weiterer Gegenstand der vorliegenden Erfindung ist ein automatisches Analysegerät mit mindestens einer Pipettiervorrichtung, einer photometrischen oder nephelometrischen Messstation und einer Datenverabeitungseinheit, dadurch gekennzeichnet, dass das Analysegerät ferner einer Steuereinrichtung umfasst, die so konfiguriert ist, dass sie ein Verfahren zur Bestimmung der Fibrinogenkonzentration gemäß der Clauss-Methode mit den folgenden Schritten steuert:
- Bereitstellen eines Reaktionsansatzes enthaltend eine Plasmaprobe und Thrombin,
- Messen der Extinktionswerte des Reaktionssatzes über die Zeit,
- Ermitteln der maximalen Extinktionsänderung und
- Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.

Derartige automatische Analysegeräte werden routinemäßig in der Analytik, der Forensik, der Mikrobiologie und der klinischen Diagnostik verwendet, um eine Vielzahl von Proben in einer Vielzahl von Untersuchungsmethoden schnell, exakt und reproduzierbar zu analysieren.

Ein erfindungsgemäßes Analysegerät mit einer Datenverabeitungseinheit, die so konfiguriert ist, dass sie die Durchführung eines Clauss-Tests in Kombination mit der oben beschriebenen kinetischen Auswertung steuert, hat den Vorteil, dass es die Durchführung einer präzisen Fibrinogenbestimmung ermöglicht, wobei für die Reagenzien, die für die Bereitstellung des Reaktionsansatzes verwendet werden, insbesondere für das Thrombinreagenz, keine ungekühlten Aufbewahrungspositionen vorgesehen sein müssen.

Das Bereitstellen eines Reaktionsansatzes enthaltend die Plasmaprobe und Thrombin in dem automatischen Analysegerät wird typischerweise mit Hilfe von einer oder mehreren automatischen Pipettiervorrichtungen durchgeführt.

Die Messung der Extinktionswerte des Reaktionssatzes über die Zeit erfolgt typischerweise in der photometrischen oder nephelometrischen Messstation.

Unter einer "photometrischen Messstation" ist eine Messeinheit zu verstehen, die mindestens eine Lichtquelle und mindestens einen Lichtdetektor aufweist und die so ausgestaltet ist, dass sie die Messung der Extinktion von Licht einer bestimmten Wellenlänge in einer Probe ermöglicht. Typischerweise ist die Wellenlänge des von der Lichtquelle emittierten Lichts so gewählt, dass es von einer in der Probe nachzuweisenden Substanz, hier Fibrin, abgeschwächt (absorbiert, reflektiert oder gestreut) wird.

Unter einer "nephelometrischen Messstation" ist eine Messeinheit zu verstehen, die mindestens eine Lichtquelle und mindestens einen Lichtdetektor aufweist und die so ausgestaltet ist, dass sie die Messung der Extinktion von Licht in einer Probe ermöglicht. Typischerweise ist die Anordnung von Lichtquelle und Lichtdetektor so gewählt, dass das Streulicht messbar ist, das von in der Probe nachzuweisenden Makromolekülen, beispielsweise von Partikelaggregaten, die infolge einer Analyt-abhängigen Reaktion in einem Reaktionsansatz entstehen, oder hier von Fibrin gestreut wird.

Für die Bestimmung der Fibrinogenkonzentration mit Hilfe einer Kalibrationskurve, ist die entsprechende Zuordnung von bekannten Fibrinogenkonzentrationen und maximalen Extinktionsänderungen, vorzugsweise in der Datenverabeitungseinheit gespeichert. In der Datenverarbeitungseinheit erfolgt dann auch das Ermitteln der Fibrinogenkonzentration durch Ablesen der der ermittelten maximalen Extinktionsänderung des Reaktionsansatzes entsprechenden Fibrinogenkonzentration.

Das nachfolgende Beispiel ist zur Veranschaulichung der Erfindung nicht aber als Einschränkung anzusehen.

### BEISPIEL

### Durchführung der Fibrinogenmethode nach Clauss

Es wurden 1:12,5-Verdünnungen von humanen Plasmaproben im Fibrinogenkonzentrationsbereich von ca. 1 g/L bis ca. 5 g/L in Owren's Veronal Puffer hergestellt. 100 µL dieser verdünnten Proben wurden bei +37 °C temperiert und 50 µL temperiertes Thrombinreagenz (ca. 100 IU/mL) wurden zugefügt. Die Reaktionsansätze wurden gemischt, und die Extinktion wurde photometrisch mit Licht einer Wellenlänge von 405 nm über einen Zeitraum von etwa 80 Sekunden gemessen. Die Bereitstellung der Reaktionsansätze sowie die Messung der Extinktion wurden in einem automatischen Analysegerät durchgeführt.

### Erfindungsgemäße Auswertung der Reaktionskinetiken

Die Auswertung der Reaktionskinetiken, also der über die Zeit gemessenen Extinktionswerte, erfolgte erfindungsgemäß, indem die maximale Extinktionsänderung einer Reaktionskinetik mittels eines Regressionsverfahrens ermittelt wurde.

Auf dieselbe Art wurde eine Kalibrationskurve mit Plasmaproben bekannter Fibrinogenkonzentrationen und den dazugehörigen, erfindungsgemäß ermittelten maximalen Extinktionsänderungen erstellt.

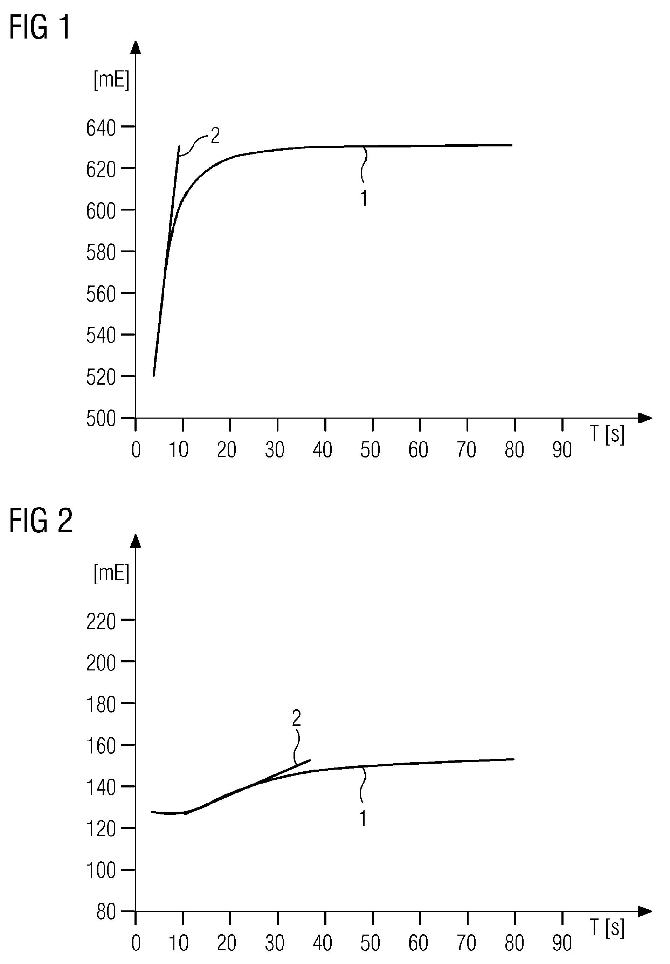

FIG. 1 zeigt den zeitlichen Verlauf der Extinktionsmesswerte (mE), also die Reaktionskinetik (1), einer Plasmaprobe mit einer hohen Fibrinogenkonzentration (ca. 5 g/L). Die Reaktion, also die Fibrinbildung, hat bereits beim Einsetzen des Reaktionsgefäßes in den Messkanal (ca. 3-4 Sekunden nach Reagenzzugabe) begonnen. Hier kann eine klassische Bestimmung der Gerinnungszeit mittels der Signaldifferenz-Methode schon nicht mehr exakt durchgeführt werden, da das Startsignal unbekannt ist. Die erfindungsgemäße Bestimmung der maximalen Reaktionsgeschwindigkeit mittels eines angepassten Regressionsintervalls ist hier die Methode der Wahl. Das angepasste Regressionsintervall ist hier sehr kurz (2 Sekunden). Die Gerade (2) ist die Gerade der maximalen Reaktionsgeschwindigkeit (Extinktionsänderung).
FIG. 2 zeigt den zeitlichen Verlauf der Extinktionsmesswerte (mE), also die Reaktionskinetik (1), einer Plasmaprobe mit einer niedrigen Fibrinogenkonzentration (ca. 1 g/L). Hier geschieht die Umwandlung von Fibrinogen in Fibrin sehr langsam und teilweise ohne klar ausgeprägtes Endplateau. In diesem Fall ist ebenfalls die Bestimmung der Reaktionsgeschwindigkeit die Methode der Wahl. Das angepasste Regressionsintervall ist hier verlängert (9 Sekunden). Die Gerade (2) ist die Gerade der maximalen Reaktionsgeschwindigkeit (Extinktionsänderung).

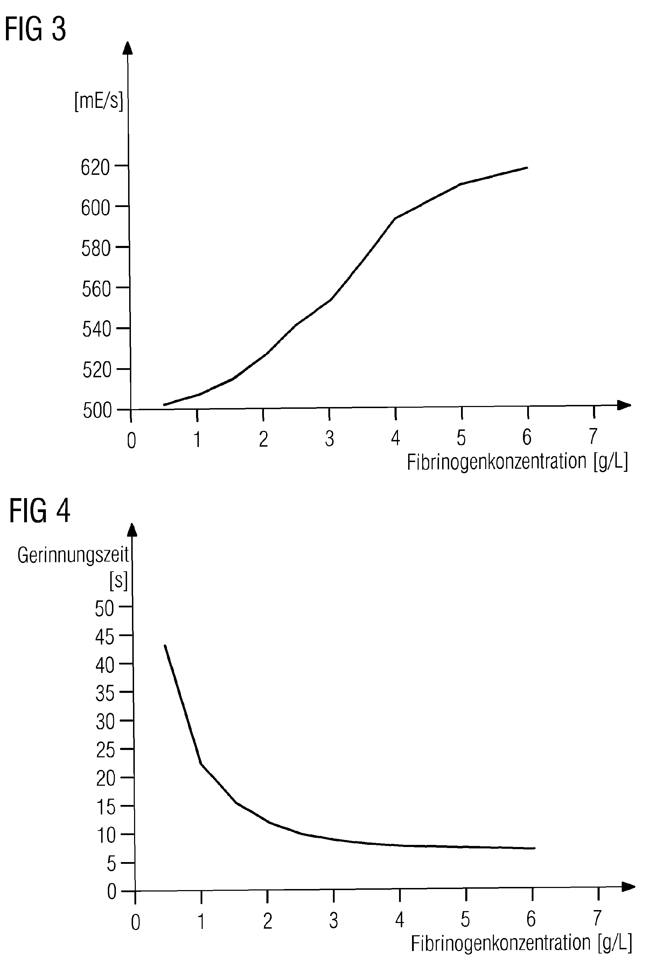

FIG. 3 zeigt eine Kalibrationskurve für die erfindungsgemäße Bestimmung der Fibrinogenkonzentration. Es wurden 10 Plasmaproben bekannter Fibrinogenkonzentration zwischen 0,5 und 6,0 g/L mit dem erfindungsgemäßen Verfahren analysiert. Es ergibt sich in allen Intervallen eine gute Spreizung zwischen den Stützpunkten. Durch eine Änderung der Probenverdünnung, z.B. als 1:4,2-Verdünnung von Proben mit einer Fibrinogenkonzentration von weniger als 1,0 g/L oder einer 1:25-Verdünnung von Proben mit einer Fibrinogenkonzentration von mehr als 6,0 g/L ließe sich der messbare Fibrinogenkonzentrationsbereich entsprechend erweitern.

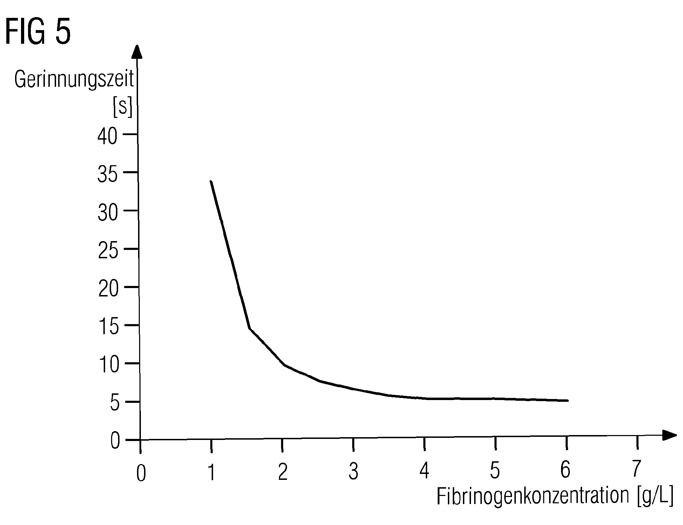

Zum Vergleich sind in den FIG. 4 und 5 Kalibrationskurven für die bisher üblichen Fibrinogenbestimmungsmethoden nach Clauss mit einer Auswertung der Gerinnungszeit über Schwellenwerte (FIG. 4) oder über die Signaldifferenz in Prozent (FIG. 5) gezeigt. In beiden Fällen ist der Verlauf der Kalibrationskurve oberhalb von etwa 4 g/L bereits so flach, dass eine sinnvolle Auswertung der Rohwerte praktisch nicht mehr möglich ist. Proben mit einer Fibrinogenkonzentration von mehr als 4 g/L müssten also bereits stärker verdünnt und nochmals gemessen werden. Der Grund für die starke Abflachung liegt unter anderem in der bereits gestarteten Reaktion zum Zeitpunkt der ersten Messwertaufnahme.

Darüber hinaus ist eine Auswertung von mit der Schwellenwert-Methode bestimmten Gerinnungszeiten bei einer Konzentration von 0,5 g/L nicht möglich, da das Signal die Mindestschwelle nicht erreicht. Eine Reduktion des Schwellenwertes als mögliche Lösung bringt die Gefahr von Falschauswertungen auf Grund von Überlagerungen in der Reaktionskurve (z.B. durch Luftblasen) mit sich.

## Patentansprüche

1. Verfahren zur Bestimmung der Fibrinogenkonzentration in einer Plasmaprobe gemäß der Clauss-Methode, das Verfahren umfassend die Schritte:
• Bereitstellen eines Reaktionsansatzes enthaltend die Plasmaprobe und Thrombin,
• Messen der Extinktionswerte des Reaktionssatzes über die Zeit,
• Ermitteln der maximalen Extinktionsänderung und
• Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.

2. Verfahren gemäß Anspruch 1, wobei das Ermitteln der maximalen Extinktionsänderung die folgenden Schritte umfasst:
• Erstellen einer Reaktionskurve aus den gemessenen Extinktionswerten über die Zeit, und
• Durchführen eines Regressionsverfahrens.

3. Automatisches Analysegerät mit mindestens einer Pipettiervorrichtung, einer photometrischen oder nephelometrischen Messstation und einer Datenverarbeitungseinheit, **dadurch gekennzeichnet, dass** das Analysegerät ferner einer Steuereinrichtung umfasst, die so konfiguriert ist, dass sie ein Verfahren zur Bestimmung der Fibrinogenkonzentration in einer Plasmaprobe gemäß der Clauss-Methode mit den folgenden Schritten umfasst:
• Bereitstellen eines Reaktionsansatzes enthaltend die Plasmaprobe und Thrombin,
• Messen der Extinktionswerte des Reaktionssatzes über die Zeit,
• Ermitteln der maximalen Extinktionsänderung und
• Bestimmen der Fibrinogenkonzentration mit Hilfe einer Kalibrationskurve, die aus einer Zuordnung von bekannten Fibrinogenkonzentrationen und maximalen Extinktionsänderungen erstellt wurde.
